# EUROPEAN PATENT SPECIFICATION

(11) **EP 0 593 684 B1**
(45) Date of publication and mention of the grant of the patent: **16.07.1997**
(21) Application number: 92918572.6
(22) Date of filing: 06.08.1992
(51) Int. Cl.: C08F 283/12, C08G 77/06, C08G 77/12, C08G 77/20, C08J 3/24, G02B 1/04

(54) **HIGH REFRACTIVE INDEX SILICONE COMPOSITIONS**
SILOXANZUSAMMENSETZUNG MIT HOHEM BRECHUNGSINDEX
COMPOSITIONS DE SILICONE A INDICE DE REFRACTION ELEVE

(30) Priority: 21.04.1992 US 871335
(43) Date of publication of application: 27.04.1994
(73) Proprietor: KABI PHARMACIA OPHTHALMICS, INC., Monrovia, CA 91017-7136 (US)
(72) Inventor: ZHOU, Stephen, Q., San Gabriel, CA 91776 (US); SY, Jennifer, C., Rancho Cucamonga, CA 91730 (US); BERTEIG, Michelle, A., Monrovia, CA 91016 (US); RICHARDS, Thomas, P., Los Angeles, CA 90068 (US)
(74) Representative: Dealtry, Brian
(86) International application number: US9206781
(87) International publication number: WO9321245

(56) References cited:
- EP-A- 0 293 560
- DE-A- 2 041 633
- US-A- 3 635 743
- US-A- 3 996 189
- US-A- 4 868 251
- US-A- 4 929 669
- US-A- 5 082 886

## Description

The present invention is generally related to curable silicone compositions. More particularly, the present invention is related to organosiloxane copolymer compositions which cure to an elastomer having superior optical resolution, excellent folding recovery and high refractive indices, making them particularly suitable for fabricating foldable intraocular lenses.

US 4,929,669 relates to organosiloxane compositions that can be cured by a platinum-catalysed hydrosilation reaction to form elastomers.

US 5,082,886 relates to a liquid moldable silicone elastomer having low compression set and resistance to fuels and oil.

Silicones are used extensively for applications in which optical quality materials having resilient characteristics are preferred. A particularly suitable application for optically clear silicone elastomers is in the fabrication of lenses which are surgically implanted in the eye as a replacement for the natural lens. Known as intraocular lenses, these are most frequently implanted subsequent to cataract surgery which results in the extraction of the natural lens. Advantageously, silicone is a suitably inert material and is well tolerated in the eye. Moreover, when carefully formulated, silicone based compositions can be prepared which have suitable mechanical strengths and sufficiently high optical resolution for use as a human prosthetic lens.

Intraocular lens implant procedures involve preparing an incision in the eye, passing the lens into the eye, and then suitably anchoring the lens within the eye so that it does not subsequently become dislodged. Procedures in which non-resilient lenses are implanted, for example, polymethylmethacrylate intraocular lenses, require incisions which are at least as long as the lens diameter or 6 - 8 mm. During the past decade small incision surgery has become increasingly popular among ophthalmic surgeons. These techniques involve implanting foldable intraocular lenses through incisions as little as 2 mm. Because silicone elastomer intraocular lenses can be folded or rolled into configurations having a reduced cross-sectional diameter, these types of lenses have grown in popularity.

Foldable intraocular lenses are preferably as thin as possible to provide reduced folded cross-sectional areas. Thinner lenses are also much easier to fold or roll into a configuration which is easily inserted. A problem associated with silicone based intraocular lenses is the inherently low refractive index of the most common and strongest polyorganosiloxane, polydimethylsiloxane (n = 1.40). As a result, lenses prepared from such materials having a low refractive index are thicker than lenses having the same degree of sight correction prepared from a higher refractive index material. Accordingly, optically effective polydimethylsiloxane based intraocular lenses are typically too thick for conveniently folding or rolling into shapes having minimum cross-sectional area.

In order to fabricate thinner foldable silicone intraocular lenses some lens manufacturers have provided silicone compositions with increased refractive indices. For example, a higher refractive index silicone has been prepared by replacing at least part of the dimethylsiloxane with the much weaker diphenylsiloxane which has a much higher refractive index. These silicones must be reinforced with a suitable filler to provide sufficient strength to the formulation. Moreover, in order for the polyorganosiloxanes of dimethylsiloxane and diphenylsiloxane to provide optically clear cured elastomers, the crosslinking reagent must match the refractive index of the copolyorganosiloxane. Typical organosiloxane crosslinking reagents are copolymers of methylhydrogensiloxane and dimethylsiloxane. These reagents, however, have low refractive indices, and when utilized in the cure of diphenylsiloxane copolymers the final cured elastomer is hazy or even opaque.

One crosslinking reagent having an acceptable refractive index is a terpolymer of dimethylsiloxane, diphenylsiloxane and methylhydrosiloxane. When utilized as a vulcanization crosslinking reagent for polydimethyldiphenylsiloxane, the resulting silicone elastomer is optically clear. However, major problems associated with intraocular lenses prepared from these elastomers are poor optical resolution and the inability for these lenses to recover sufficient optical resolution once they are unfolded from a folded configuration. Thus optical clarity associated with a particular material does not necessarily guarantee good optical resolution and folding recovery.

Additionally, silicone based intraocular lenses are typically prepared with silicone based polyorganosiloxanes having a significant amount of low molecular weight material. The presence of the small molecular weight silicones can be responsible for significant lens shrinkage and weight loss.

Accordingly, it is an objective of the present invention to provide a curable optically clear silicone composition having a high refractive index.

It is also an objective of the present invention to provide a curable silicone composition useful for fabricating intraocular lenses having a low folded profile.

It is further an objective of the present invention to provide a curable silicone composition useful for fabricating intraocular lenses having superior optical resolution and folding recovery.

The present invention provides curable polyorganosiloxane compositions as defined in the accompanying claims. The compositions can vulcanize to optically clear silicone elastomers having refractive indices of at least 1.46. Since the curable polyorganosiloxane compositions of the present invention additionally demonstrate superior optical resolution characteristics and near 100% post-folding optical resolution recovery, they are particularly suitable for fabricating foldable intraocular lenses used in small incision intraocular lens implant surgery.

The high refractive index, curable polyorganosiloxane compositions include at least two vinyl terminated polyorganosiloxane copolymers, each of which is from about 80 mole % to about 95 mole % dimethylsiloxane and from about 5 mole % to about 20 mole % diphenylsiloxane. The first vinyl terminated polyorganosiloxane is present at a concentration of from about 30% to about 55% and has a molecular weight range sufficient to provide a first vinyl terminated polyorganosiloxane copolymer viscosity of from about 400 cps to about 2500 cps. The second vinyl terminated polyorganosiloxane is present at a concentration of from about 45% to about 70% and has a molecular weight range sufficient to provide a second vinyl terminated polyorganosiloxane viscosity of from about 2500 cps to about 9500 cps.

Additionally, the polyoganosiloxane compositions of the present invention include the organohydrosiloxane crosslinking reagent tetrakis(dimethylsiloxane)silane which has a SiO₂ structure unit and a refractive index of 1.46 and contributes to the optical clarity of the composition and to the improved strength in the resulting cured elastomer.

Optionally, the compositions of the present invention further include at least one ultraviolet absorbing compound which is capable of absorbing significant amounts of ultraviolet radiation having wavelengths from about 200 nm to about 405 nm. Preferably, the ultraviolet absorbing compound is capable of co-polymerizing with the organohydrogensiloxane crosslinking reagent and vinyl terminated polyorganosiloxane base resins. This capability allows the ultraviolet absorbing compound to covalently bind with the elastomer and precludes its mass transfer from the cured composition. Exemplary embodiments of the present invention also include ultraviolet absorbing compounds complexed with the multi-functional crosslinking reagent.

The high refractive index compositions of the present invention can be cured to polyorganosiloxane elastomers using conventional platinum hydride cure techniques. Preferably, suitable silicone elastomer molding techniques are utilized to form molded objects, such as foldable, high refractive index intraocular lenses. The resulting molded elastomer maintains the highly superior optical clarity present in the uncured composition, and furthermore exhibits superior folding recovery. This important characteristic is attributed to the bimodal molecular weight ranges of the polyorganosiloxanes which cure to form strong interconnecting polymer networks. Thus, intraocular lenses fabricated from the polyorganosiloxane compositions of the present invention have smaller profiles and maintain high optical resolution after having been folded, making them particularly useful in small incision intraocular lens implant surgery.

Further objects, features and advantages of the curable high refractive index polyorganosiloxane compositions of the present invention, as well as a better understanding thereof, will be afforded to those skilled in the art from a consideration of the following detailed explanation of preferred exemplary embodiments.

### DETAILED DESCRIPTION OF EXEMPLARY EMBODIMENTS

The present invention provides high refractive index, curable polyorganosiloxane compositions which cure to polyorganosiloxane elastomers having superior optical clarity, highly improved optical resolution, and near 100% post-folding optical resolution recovery.

Because the compositions of the present invention are elastomeric and have refractive indices on the order of 1.46, they are useful for fabricating thin profile optical lenses which advantageously can be folded or rolled to a small profile. In particular, the combination of high refractive index and superior post-folding optical resolution recovery make the curable organosiloxane compositions of the present invention particularly suitable for the fabrication of intraocular lenses used in small incision implant procedures. However, the utility of the compositions of the present invention is not limited to intraocular lenses, but includes applications in which the combination of physical and optical characteristics of the cured polyorganosiloxane compositions are desirable. Accordingly, it is contemplated to be within the scope of the present invention to additionally provide compositions for corneal contact lenses and intrastromal lenses.

In a broad aspect, the present invention provides curable silica filled polyorganosiloxane compositions of at least two vinyl terminated copolymers of dimethylsiloxane and diphenylsiloxane. The polyorganosiloxane compositions have a multimodal molecular weight distribution with at least one vinyl terminated copolymer differing substantially in molecular weight from a second vinyl terminated copolymer. Once cured with a platinum or platinum containing catalyst and crosslinking reagent or suitable polyorganohydrosiloxane, the compositions of the present invention form mechanically strong and optically superior interconnecting polymer networks.

More particularly, the curable polyorganosiloxane compositions of the present invention include a base resin of at least two vinyl terminated copolymers of from about 80 mole % to about 95 mole % dimethylsiloxane and from about 5 mole % to about 20 mole % diphenylsiloxane. At least one first vinyl terminated copolymer has a concentration of from about 30 wt% to about 55 wt% and a molecular weight sufficient to provide the first vinyl terminated copolymer with a viscosity of from about 400 cps to about 2500 cps. Similarly, at least one second vinyl terminated copolymer of dimethylsiloxane and diphenylsiloxane has a concentration of from about 45 wt% to about 70 wt% and a molecular weight sufficient to provide a second vinyl terminated copolymer with a viscosity of from about 4000 cps to about 9500 cps. The exemplary polyorganosiloxane copolymer compositions further include filler material, suitable catalyst and the organohydrogenpolysiloxane crosslinking reagent.

The vinyl terminated copolymers of dimethylsiloxane and diphenylsiloxane utilized in the compositions of the present invention have the following general formula: 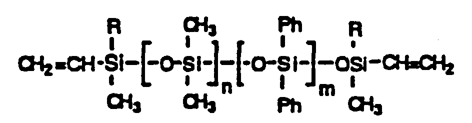 where R is a monovalent organic radical selected from the group consisting of -CH₃ and CH=CH₂. Note that the vinyl terminated copolymers can include more than one terminal vinyl functionality. Preferred copolymers are random copolymers of dimethylsiloxane and diphenylsiloxane. However, block copolymers and alternating copolymers containing dimethylsiloxane and diphenylsiloxane in a regular repeating pattern are also contemplated as being within the scope of the present invention.

The values n and m indicate the number of dimethylsiloxane units and the number of diphenylsiloxane units, respectively in the polyorganosiloxane copolymers. The at least one first vinyl terminated copolymer has a value sufficient to provide a first vinyl terminated copolymer viscosity of from about 400 cps to about 2500 cps. Similarly, the at least one second vinyl terminated copolymer has a value n + m sufficient to provide a second vinyl terminated copolymer viscosity of from about 2500 cps to about 9500 cps. Accordingly, exemplary embodiments of the present invention include first and second vinyl terminated copolymers having values of n from about 60 to about 200 and from about 200 to about 330, respectively. Similarly, these exemplary embodiments include first and second vinyl terminated copolymers having values of m from about 9 to about 35 and from about 35 to 55, respectively. Preferred exemplary embodiments have second vinyl terminated copolymer viscosities ranging from 4000 cps to 9500 cps and have values of n and m ranging from 225 to about 330 and 40 to 55, respectively. Moreover, while exemplary embodiments of the present invention include base resins of vinyl or divinyl terminated copolymers having from 5 mole % to 20 mole % diphenylsiloxane, preferred embodiments include vinyl or divinyl terminated copolymers having from 12 mole % to 18 mole % diphenylsiloxane. Most preferred embodiments have about 13 - 15 mole % diphenylsiloxane.

It is evident from the discussion above that the values n and m determine the molecular weight and viscosity of the first and second vinyl terminated copolymers. It is also apparent that the molecular weight distribution of the preferred polyorganosiloxane compositions of the present invention is bimodal. That is, the first and second vinyl terminated copolymers have distinguishably different molecular weights with little or no overlap. As will be discussed further below, this bimodal molecular weight distribution is believed to be responsible for the superior strength and excellent post folding recovery characteristic of the highly interconnected network of polymer chains in the final cured organosiloxane polymer.

The filler material utilized in the polyorganosiloxane compositions of the present invention is preferably fumed silica having an average particle size on the order of less than about 11 nanometers. In order to obtain superior optical characteristics the particle size should be as small as possible. Fumed silica having an average particle size of about 7 nanometers in diameter is particularly suitable because the small particle size does not interfere with the wavelength of visible light and contributes to an improved optical resolution in the cured composition. Fumed silica is used in amounts of from 8 phr (parts per hundred resin) to 25 phr. Commercial fumed silica with particle sizes as low a 7 nm are available from a number of sources including CABOT and Sigma.

Preferred exemplary embodiments of the present invention include from about 11 phr to 14 phr small particle size fumed silica which has been surface treated with a silazane. These silazane treated surfaces provide the fumed silica particles with a siloxane compatible surface. This characteristic improves the siloxane wettability of the fumed silica and further contributes to the superior optical qualities of the polyorganosiloxane compositions.

Preferred silazanes and methods for carrying out the fumed silica surface treatment include the in situ reaction of small particle size fumed silica with hexamethyldisilazane and/or 1,3-divinyltetramethyldisilazane. These silazanes readily react with the -OH functionalities on fumed silica, forming a trimethylsiloxane coating on the silica surface. The 1,3-divinyltetramethyldisilazane provides the added advantage of incorporating vinyl functionalities on the fumed silica surface which can covalently interact with the polyorganosiloxane copolymers during the curing process.

The crosslinking reagent used in the compositions of the present invention has multiple active hydride functionalities for reacting with vinyl functionalities in a platinum cure system; tetrakis(dimethylsiloxane)silane has four reactive hydride units per molecule. The organohydrogenpolysiloxane is available from a variety of commercial sources including Petrarch. Advantageously, this organohydrogensiloxane has a SiO₂ structure unit which makes it optically compatible with the vinyl terminated polyorganosiloxanes which make up the base resin and also have refractive indices of about 1.46. This crosslinking reagent has the capability of reacting with up to four different copolymer chains during the curing process, resulting in a tightly crosslinked interconnecting polymeric network.

Optionally, the polyorganosiloxane copolymer compositions of the present invention further include an ultraviolet (UV) absorbing compound which preferably has a high absorptivity of ultraviolet radiation for wavelengths up to about 405 nm. When incorporated in organosiloxane compositions used to fabricate optical materials utilized in ophthalmic applications, these UV absorbing compounds are a safeguard against excessive UV radiation exposure to the retina and other ocular structures. Exemplary embodiments have from about 0.1 phr to as high as 10 phr UV absorbing compound. The preferred concentration of UV absorbing compound depends upon the molar absorptivity of the UV absorbing compound, with the more high absorbing compounds being present at lower concentrations. Suitable UV absorbing compounds are those compounds having desirable absorption characteristics, minimum ocular toxicity and preferably having a sufficiently high solubility in the vinyl terminated organosiloxane copolymers. For example, any of a large variety of hydroxy benzophenones and hydroxyphenylbenzotriazoles, available from numerous commercial sources, have these physical and chemical characteristics.

In order to assure that no amount or negligible amounts of the UV absorbing compound is available to migrate to the surface of an ophthalmic lens once it is situated in the ocular environment, the UV absorbing compound preferably is capable of copolymerizing with the vinyl terminated organosiloxane copolymers utilized in the compositions of the present invention. Thus, for example, UV absorbing compounds having active vinyl functionalities and/or active silicone hydride functionalities are particularly suitable for incorporating into the vinyl terminated organosiloxane copolymers of the present invention. For example, vinyl/and or silicone hydride modified hydroxy benzophenones or hydroxy phenylbenzotriazoles incorporated in vinyl terminated organosiloxane copolymers will react with the vinyl terminated organosiloxane copolymers once a crosslinking reaction is initiated by platinum or platinum containing catalyst. More specifically, the allyloxypropyl modified hydroxy phenylbenzotriazole known as Tinuvin 326 or 2-[5-chloro-2H-benzotriazol-2-yl]-6-[1,1-dimethylethyl]-4-[-2-propenyloxypropyl]phenol is particularly suitable because of its high absorptivity at wavelengths up to 405 nm, its solubility and its vinyl functionality.

It is also contemplated as being within the scope of the present invention to provide compositions which incorporate multi-functional UV absorber compounds. In particular, UV absorbing compounds having a plurality of silicone hydride functionalities have advantageous chemical reactivity properties. Multi-functional silicone hydride UV absorbers contribute to increased crosslinking and thus result in a "tighter" interconnecting polymer network in the final cured composition. Additionally, UV absorbing compounds are bulky and somewhat sterically hindered. By providing a plurality of reactive groups on the UV absorbing compound, there is a greater probability of forming covalent bonds with the polymerizing organosiloxane copolymers. If monofunctional UV absorbers are merely mixed with a curable silicone formulation, some of the UV absorber will incorporate into the silicone network. However, a substantial amount will remain unreacted and is available for extraction or migration to the surface of a formed intraocular lens within the ocular environment. Moreover, the multi-functional nature of the UV absorbing compound does not cause undesirable "chain termination" during the curing process which results in shortened polyorganosiloxane polymer chains and a reduced interconnecting network.

Exemplary multi-functional UV absorbers can be prepared by reacting any of the vinyl modified hydroxy benzophenones or vinyl modified hydroxy phenylbenzotriazoles mentioned above with multi-functional active silicone hydrides. For example, when 2-[5-chloro-2H-benzotriazol-2yl-]-6-[1,1,2-dimethylethyl]-4-[2-propenyloxypropyl]phenol is reacted with tetrakis(dimethylsiloxane)silane, the resulting silicone hydride modified hydroxyphenylbenzotriazole has three reactive silicone hydride functionalities and can act as additional crosslinking reagent for the vinyl terminated organosiloxane copolymers. Similarly, this same hydroxy benzotriazole can be reacted with a terpolymer of dimethylsiloxane, diphenylsiloxane, and methylhydrosiloxane to form a UV absorbing compound having multiple hydride functionalities.

Preferred exemplary embodiments of the present invention further include small amounts of inhibitor, on the order of about 0.1 pmr (parts per million resin) to about 5 pmr, to extend the pot-life up to 8 hours. A wide variety of suitable inhibitors are commercially available and can be utilized in the compositions of the present invention. Specifically, 1,3,5,7-tetravinyltetramethylcyclotetrasiloxane and/or 1-octyn-3-ol provide sufficient inhibiting properties for incorporating in the compositions of the present invention.

The compositions of the present invention can be effectively cured to a crosslinked, high refractive index elastomeric material with interconnected polymer networks having superior optical properties. The cure reaction is preferably catalyzed with a platinum or platinum containing catalyst which is present at catalytic effective concentrations. Typical catalytic effective concentrations are from about 5 pmr to about 50 pmr. In addition to platinum, suitable platinum containing compounds having utility as catalysts in the compositions of the present invention include H₂PtCl₆, platinumdivinyltetramethyldisiloxane complex, and platinum-cycloydovinylmethylsiloxane complex.

The preferred vinyl terminated organosiloxane copolymers, crosslinking reagent, fumed silica, UV absorbers, inhibitor, and catalyst containing compositions of the present invention are homogeneous blends which cure to a high refractive index, elastomeric organosiloxane. The compositions are prepared utilizing polymer and prepolymer formulating and mixing techniques known in the polymer art. Typically, these techniques include accurately weighing and/or accurately measuring volumes of the desired vinyl terminated organosiloxane copolymers, fumed silica, and additives and then transferring each component to suitable mixing equipment. Then, subsequently mixing the composition until a homogeneous blend of the components is obtained which is ready for curing into the desired shaped article.

In order to prepare lenses for ophthalmic applications fabricated from the compositions of the present invention, the preferred procedure is to provide lens molds of the desired shape and then fill the molds with the above described uncured vinyl terminated organosiloxane compositions. The filled molds are then heated to a curing temperature and for a length of time sufficient to form a crosslinked interconnected polymer network. Typical curing temperatures and curing periods include from about 90°C to about 160°C and from about 1 min. to about 60 min.

Preferably, after curing the organosiloxane copolymers and forming the article or lens, the lens is extracted with a low molecular weight alcohol, such as ethyl alcohol or isopropyl alcohol. This step causes very low molecular weight and alcohol soluble organosiloxanes to extract from the lens and further provides a precautionary measure against their extraction in the ocular environment after the lens is in use. There is no detectable UV absorbing compound to be extracted because UV absorbing agent has been covalently bonded to the crosslinker.

Because the refractive index of the vinyl terminated organosiloxanes, fumed silica, and UV absorbers are very similar, each about 1.46, the resulting cured elastomers have superior optical clarity. Moreover, the multi-functional crosslinking reagents and/or UV absorbing compounds contribute to a highly crosslinked interconnecting network which is believed to be responsible for the 100% post folding recovery exhibited by the cured compositions of the present invention. This property is further enhanced by the bimodal molecular weight distribution of the vinyl terminated organosiloxane copolymers which form interconnecting long and short polymer chains. The combined effect of the bimodal network and the tightly crosslinked network is a superior "memory" characteristic in the cured elastomer. This "memory" characteristic is exemplified by the cured elastomer's ability to recover its original dimensions and its optical resolution after deformation.

The exceptional optical and physical characteristics of the cured compositions of the present invention as well as exemplary compositions and procedures for their preparation are further illustrated by the following nonlimiting examples.

### Example 1 (not according to the present invention)

### Procedure For Preparing Terpolymer Crosslinking Reagent

A terpolymer of dimethylsiloxane, diphenylsiloxane, and methylhydrosiloxane was prepared by first combining 81 grams of hexamethyldisiloxane, 323 grams of octamethylcyclotetrasiloxane and a catalytic amount of triflic acid and allowing the reactants to polymerize at 60°C for 16 hours. This reaction resulted in a yield of 354 grams of a 10 cps polydimethylsiloxane having trimethylsilyl end groups which was then allowed to equilibrate with 180 grams of octaphenylcyclotetrasiloxane in the presence of potassium silanoate catalyst at 140° - 150°C for 48 hours. This equilibration procedure resulted in a copolymer of dimethylsiloxane and diphenylsiloxane with a viscosity of 60 cps and a refractive index of 1.465. Then 468 grams of this copolymer was reacted with 172 grams of 1,3,5,7-tetrahydrotetramethylcyclotetrasiloxane in the presence of triflic acid catalyst at 50°C for 3 days to provide a terpolymer having 10 dimethylsiloxane units, 2 diphenylsiloxane units, and 10 hydroxymethylsiloxane units.

### Example 2 (not according to the present invention)

### Cured Organosiloxane with UV Absorber

A curable high refractive index polyorganosiloxane composition was obtained by first preparing a base resin of 58 parts vinyl terminated dimethyldiphenylsiloxane having a viscosity of 2,500 cps and 42 parts vinyl terminated dimethyldiphenylsiloxane having a viscosity of 4,900 cps. Then 12 phr (parts per hundred parts resin) of fumed silica, 25 pmr (parts per million parts resin) suitable inhibitor, 0.5 phr Tinuvin 326 (UV absorber), 15 pmr platinum catalyst and 5 phr of the terpolymer prepared in Example 1 were mixed with the base resin to form a homogeneous mixture. The mixture was poured into molds suitable for preparing intraocular lenses and slabs having dimensions of 3 inch x 4 inch, cured at 130° - 140°C for 45 - 60 minutes, and post cured at 110°C for 16 hours.

The resulting cured slabs were extracted in ethanol for 24 hours at 25°C. Following this extraction step a variety of physical and optical tests were performed including weight loss after extraction, tensile strength, elongation at break, and Shore hardness. The prepared intraocular lenses were tested for optical folding recovery at both low and high diopters resolution, and the wavelength at which the ultra-violet cutoff occurs. Table I illustrates the results of these tests.

**Table I**

| | |
|---|---|
| Weight Loss after extraction | <4.5 wt% |
| Tensile strength | 400 psi |
| Elongation at Break | 350% |
| Shore Hardness | 29 |
| Optical efficiency post folding (30 diopter lens) | |
| in air | 64% |
| in aqueous | 85% |
| Optical efficiency post folding (15 diopter lens) | |
| in air | 90% |
| in aqueous | 90% |
| Ultra-violet cut off | 400 nm |

### Example 3

### Cured Organosiloxane Without UV Absorbing Compound

A curable high refractive index organosiloxane composition was obtained by first preparing a base resin of 58 parts vinyl terminated dimethyldiphenylsiloxane having a viscosity of 400 cps and 42 parts vinyl terminated dimethyldiphenylsiloxane having a viscosity of 9,500 cps. Then 12 phr (parts per hundred parts resin) of fumed silica, 25 pmr (parts per million parts resin) suitable inhibitor, 2.35 phr tetrakis(dimethylsiloxane)silane and 15 pmr platinum catalyst were mixed with the base resin to form a homogeneous mixture. The mixture was poured into molds suitable for preparing intraocular lenses and slabs having dimensions of 3 inch x 4 inch , cured at 130° - 140°C for 45 - 60 minutes, and post cured at 110°C for 16 hours.

The resulting cured slabs were extracted in ethanol for 24 hours at 25°C. Following this extraction step a variety of physical and optical tests were performed including weight loss after extraction, tensile strength, elongation at break, and Shore hardness. The prepared intraocular lenses were tested for optical folding recovery at both low and high diopters resolution, and the wavelength at which the ultra-violet cutoff occurs. The results obtained from these tests were similar to those reported in Table I above for samples containing UV absorbing compound.

### Example 4

### Preparation of UV Absorbing Compound with Multiple Hydrides

In order to prepare a UV absorbing compound having a plurality of silicone hydride functionalities, a mixture of 14.7 grams of the vinyl modified hydroxy benzotriazole, (2-[5-chloro-2H-benzotriazol-2-yl]-6-[1,1-dimethylethyl]-4-[2-propenyloxypropyl]phenol), 100 grams of tetrakis(dimethylsiloxane)silane, and 2µl of platinum catalyst H₂PtCl₆, was stirred at 70°C - 80°C for 48 hours under an inert gas atmosphere. The product obtained from this reaction was analyzed by ¹H NMR to determine the presence of vinyl protons which would indicate incomplete conversion of the vinyl functionalities to silicone hydride. The ¹H NMR results showed no detectable vinyl protons indicating complete hydrosilylation conversion to silicone hydride. It should be noted that the mixture containing the UV absorbing compound prepared above can be utilized as the source of UV absorbing compound and crosslinker for the copolymer reaction mixture. In the reaction described above, 14.7 grams (0.0368 mole) of the UV absorber was reacted with 100 grams (0.304 mole) of the crosslinker tetrakis(dimethylsiloxane)silane. There are 4 Si-H functional groups in each crosslinker while there is only one vinyl functional group in each UV absorber. Thus, the mole ratio of the crosslinker to the UV absorber is 8/1 and only one Si-H group out of 8 crosslinker molecules will react with one UV absorber molecule. The resulting mixture contains the crosslinker tetrakis(dimethylsiloxane)silane and the UV-crosslinker which has three Si-H functional groups available for crosslinking a silicone network. The ratio of the crosslinker to UV absorber-crosslinker in the final mixture is about 7/1.

### Example 5

### Cured Polyorganosiloxane Composition With UV Absorbing Compound

A curable high refractive index polyorganosiloxane composition was obtained by first preparing a base resin of 52 parts vinyl terminated dimethyldiphenylsiloxane having a viscosity of 4,900 cps and 48 parts vinyl terminated dimethyldiphenylsiloxane having a viscosity of 700 cps. Then 13 phr (parts per hundred parts resin) of fumed silica, 25 pmr (parts per million parts resin) suitable inhibitor, 1.85 phr of the multi-functional UV absorbing compound prepared in Example 4 above and 15 pmr platinum catalyst was mixed with the base resin to form a homogeneous mixture. The mixture was poured into molds suitable for preparing intraocular lenses and slabs having dimensions of 3 inch x 4 inch , cured at 130° - 140°C for 45 - 60 minutes, and post cured at 110°C for 16 hours.

The resulting cured slabs were extracted in ethanol for 24 hours at 25°C. Following this extraction step a variety of physical and optical tests were performed including tensile strength and tear strength. The 30 diopter intraocular lenses were tested for optical folding recovery and resolution. The 30 diopter lenses made from this formulation showed excellent folding recovery and optical resolution. Table II illustrates the results of additional tests.

**Table II**

| | |
|---|---|
| Tensile strength | 480 psi |
| Tear strength | 25 pli |

### Example 6

### Properties of IOLs Prepared From Organosiloxane Compositions of the Present Invention Compared With Commercial IOLs.

A curable high refractive index polyorganosiloxane composition was obtained by first preparing a base resin of 52 parts vinyl terminated dimethyldiphenylsiloxane having a viscosity of 5000 cps and 48 parts vinyl terminated dimethyldiphenylsiloxane having a viscosity of 700 cps. Then 13 phr (parts per hundred parts resin) of fumed silica, 25 pmr (parts per million parts resin) suitable inhibitor, 2 phr of UV absorbing compound prepared as in Example 4, and 15 pmr platinum catalyst was mixed with the base resin to form a homogeneous mixture. The mixture was injected into molds suitable for preparing intraocular lenses and cured at 110° - 140°C for 1 - 10 minutes, and post cured at 110°C for 2 hours.

The resulting IOLs were extracted in ethanol for 24 hours at 25°C. Following this extraction step a variety of physical and optical tests were performed on the IOLs. The same tests were also performed on two commercial silicone elastomer, foldable IOLs, identified as commercial A and commercial B. Table III illustrates the results of these tests.

**TABLE III**

| TEST | COMMERCIAL A | COMMERCIAL B | IOL OF PRESENT INVENTION |
|---|---|---|---|
| Curability | 10 min @ 130°C | 10 min @ 130°C | 10 min @ 130°C |
| Refractive Index | 1.4130 ± 0.0005 | 1.4127 ± 0.0005 | 1.4629 ± 0.0005 |
| Weight Loss | 4.1% ± 0.1% | 5.2% ± 0.1% | 3.0% ± 0.1% |
| Overall Shrinkage | 4.5% ± 0.2% | 4.3% ± 0.2% | 3.0% ± 0.5% |
| Tensile Strength | 513 ± 40 [PSI] | 842 ± 70 [PSI] | 500 ± 50 [PSI] |
| Elongation | 484% ± 40% | 163% ± 10% | 495% ± 40% |
| Tear Resistance | 42 + 4 [PLI] | 16 + 2 [PLI] | 25 + 3 [PLI] |
| Pre-Folding Resolution Efficiency | 72%±7%[Lo Diop.] 64%±7%[Hi Diop.] | 72%±7%[Lo Diop.] 64%±7%[Hi Diop.] | >90%[Lo Diop.] 80%[Hi Diop] |
| Post-Folding Resolution Efficiency | 72%±7%[Lo Diop.] 64%±7%[Hi Diop.] | 72%±7%[Lo Diop.] 64%±7%[Hi Diop.] | >90%[Lo Diop.] 72%±7%[Hi Diop] |
| Folding Recovery | 100% | 100% | 100% |
| Autoclavability | Yes | Yes | Yes |
| Durometer* | 31 ± 4 | 45 ± 3 | 29 ± 1 |
| Specific Gravity | 1.05 ± 0.02 | 1.07 ± 0.02 | 1.01 ± 0.02 |
| UV Cut-Off | | | 400.2nm@95%abs [2mm thickness] |

| | | | |
|---|---|---|---|
| * Lower durometer reading will cause the minimum "spring" effect during the lens unfolding process. | | | |

### Example 7

Polyorganosiloxane copolymers having utility in the curable high refractive index organosiloxane compositions of the present invention were prepared as follows:

A vinyl terminated end blocker was prepared by adding 2950 grams of octamethylcyclotetrasiloxane (D₄), purchased from Petrarch, 500 grams of 1,3-divinyltetramethyldisiloxane and 15 grams of tetramethylammonium hydroxide pentahydrate to a 5 L 3-neck round bottom flask equipped with a magnetic stirring bar, a reflux condenser, and a thermometer. The components were reacted at 80° - 100° C for 72 hours while stirring under constant positive argon atmosphere. The reacted mixture was allowed to cool and its viscosity was determined to be 45 cps.

The reacted mixture was worked-up by adding 250 mL of petroleum ether and 400 mL of water for every 1000 mL of mixture. Then the mixture was washed with dilute HCl (0.01 N) aqueous solution in a 2 L separatory funnel until the pH of the washings was 6.0 - 6.5. Following the dilute acid wash, the mixture was washed with three separate portions of fresh water. The organic layer was drained into a crystallizing dish and dried in a vacuum oven at 60 °C.

Vinyl terminated polyorganosiloxane copolymers were prepared by adding 768 grams of D₄, 336 grams of octaphenylcyclotetrasiloxane (D₄^{Ph}) and 48.6 grams of the end blocker prepared above to a 2L round bottom flask equipped with a magnetic stirring bar, a reflux condenser and a thermometer. The mixture was heated to 140°C in an oil bath and 0.2 grams of potassium silanoate was added while stirring under argon. The reaction was stirred for 3 days at 140° C and at the end of the third day, the viscosity was measured to be 4900 cps at room temperature. On the fourth day no further viscosity changes were observed. After cooling to room temperature, the mixture was extracted with 10% tetrahydrofuran in ethanol 4 times, dried under vacuum and filtered through a Whatman ashless filter paper in a Gelman stainless steel filter under 90 psi.

A vinyl terminated polyorganosiloxane copolymer having a viscosity of 700 cps was prepared in a manner similar to that above except 654 grams of D₄, 319 grams of D₄^{Ph}, and 145.5 grams of end blocker were utilized in the reaction.

Having thus described exemplary embodiments of the present invention, it should be noted by those skilled in the art that the disclosures herein are exemplary only and that alternatives, adaptations and modifications may be made within the scope of the present invention.

The unit of viscosity referred to above ie centipoise can be converted to the SI unit Pascal-second (Pa.s.) by multiplying by 0.001.

## Claims

1. A high refractive index, curable polyorganosiloxane composition useful for fabricating intraocular lenses, said composition comprising:
from about 30 wt% to about 55 wt% of a first vinyl terminated copolymer resin having from about 80 mole % to about 95 mole % dimethylsiloxane and from about 5 mole % to about 20 mole % diphenylsiloxane, said first vinyl terminated copolymer having a molecular weight sufficient to provide a first vinyl terminated copolymer viscosity of from about 0.4 Pa.s (400 cps) to about 2.5 Pa.s. (2500 cps);
from about 45 wt% to about 70 wt% of a second different vinyl terminated copolymer resin having from about 80 mole % to about 95 mole % dimethylsiloxane and from about 5 mole % to about 20 mole % diphenylsiloxane, said second vinyl terminated copolymer having a molecular weight sufficient to provide a second vinyl terminated copolymer viscosity of from about 2.5 Pa.s. (2500 cps) to about 9.5 Pa.s. (9500 cps);
organohydrogenpolysiloxane crosslinking reagent of tetrakis(dimethylsiloxy)silane; and
from about 8 to about 25 parts fumed silica filler per one hundred parts of resin.

2. The high refractive index, curable polyorganosiloxane composition of claim 1 further including platinum catalyst.

3. The high refractive index, curable polyorganosiloxane composition of claim 1 or 2 wherein said first vinyl terminated polysiloxane has the formula: 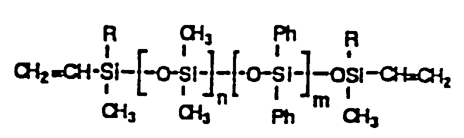 where R is a monovalent organic radical selected from the group consisting of -CH₃, and CH=CH₂ and n + m is an integer sufficient to provide said first vinyl terminated copolymer viscosity of from about 0.4 Pa.s (400 cps) to about 2.5 Pa.s. (2500 cps).

4. The high refractive index, curable polyorganosiloxane composition of any of claims 1 to 3 wherein said second vinyl terminated polysiloxane has the formula: 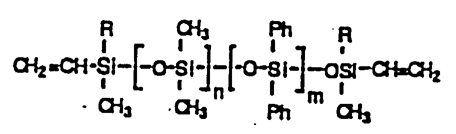 wherein R is a monovalent organic radical selected from the group consisting of -CH₃ and CH=CH₂ and n + m is an integer sufficient to provide said second vinyl terminated copolymer viscosity of from about 4.0 Pa.s. (4000 cps) to about 9.5 Pa.s. (9500 cps).

5. The high refractive index curable polyorganosiloxane composition of any one of claims 1 to 4 wherein said first vinyl terminated polysiloxane is from about 12 mole % to about 18 mole % diphenylsiloxane.

6. The high refractive index, curable polyorganosiloxane composition of any one of claims 1 to 5 wherein said second vinyl terminated polysiloxane is from about 12 mole % to about 18 mole % diphenylsiloxane.

7. The high refractive index, curable polyorganosiloxane composition of any one of claims 1 to 6 wherein said first vinyl terminated polysiloxane viscosity is about 0.7 Pa.s (700 cps).

8. The high refractive index, curable polyorganosiloxane composition of any one of claims 1 to 7 wherein said second vinyl terminated polysiloxane viscosity is about 5.0 Pa.s. (5000 cps).

9. The high refractive index curable, polyorganosiloxane composition of any one of claims 1 to 8 wherein said fumed silica has an average particle diameter of about 7 nanometers.

10. The high refractive index curable, polyorganosiloxane composition of any one of claims 1 to 9 further including an ultra-violet absorbing compound.

11. The high refractive index, curable polyorganosiloxane composition of claim 10 wherein said ultra-violet absorbing compound is vinyl hydroxyphenylbenzotriazole.

12. The high refractive index, curable polyorganosiloxane composition of claim 10 wherein said ultraviolet absorbing compound is silicone hydride hydroxyphenylbenzotriazole.

13. A high refractive index, curable polyorganosiloxane composition useful for fabricating intraocular lenses, said composition comprising:
a base resin of:
about 48 wt% of a first vinyl terminated copolymer having from about 82 mole % to about 88 mole % dimethylsiloxane and from about 12 mole % to about 18 mole % diphenylsiloxane, said first vinyl terminated copolymer having a molecular weight sufficient to provide a first vinyl terminated copolymer viscosity of about 0.7 Pa.s. (700 cps), and
about 52 wt% of a second different vinyl terminated copolymer having from about 82 mole % to about 88 mole % dimethylsiloxane and from about 12 mole % to about 18 mole % diphenylsiloxane, said second vinyl terminated copolymer having a molecular weight sufficient to provide a second vinyl terminated copolymer viscosity of about 5.0 Pa.s. (5000 cps);
from about 11 to about 14 parts per hundred base resin of fumed silica;
from about 5 to about 50 parts per million base resin of platinum containing catalyst;
from about 1.5 to about 5 parts per hundred base resin organohydrogenpolysiloxane crosslinking reagent of tetrakis (dimethylsiloxane) silane; and
from about 0.1 to about 2 parts per hundred resin of ultra-violet absorbing compound selected from the group consisting of hydroxybenzophenones and hydroxyphenylbenzotriazoles.

14. The high refractive index, curable polyorganosiloxane composition of claim 13 wherein said fumed silica is surface treated with compounds selected from the group consisting of hexamethyldisilazane and 1,3-divinyltetramethyldisilazane.

15. The high refractive index, curable polyorganosiloxane composition of claim 13 or 14 wherein said vinyl hydroxybenxotriazole is 2-[5-chloro-2H-benzotriazol-2-yl]-6-[1,1-dimethylethyl]-4-[2-propenyloxypropyl] phenol.

16. The high refractive index, curable polyorganosiloxane composition of the claim 15 wherein said 2-[5-chloro-2H-benzotriazol-2-yl]-6-[1,1-dimethylethyl]-4-[2-propenyloxypropyl] phenol is hydrosilylated with tetrakis (dimethylsiloxy) silane.

17. The high refractive index, curable polyorganosiloxane composition of the claim 15 wherein said 2-[5-chloro-2H-benzotriazol-2-yl]-6-[1,1-dimethylethyl]-4-[2-propenyloxypropyl]phenol is hydrosilylated with a terpolymer of diphenylsiloxane, dimethylsiloxane and methylhydrogensiloxane.

18. The high refractive index, curable polyorganosiloxane composition of any one of claims 13 to 17 wherein said fumed silica has an average particle size of about 7 nanometers.

19. An elastomeric, optically clear, high refractive index lens having superior post-folding optical resolution recovery, said lens comprising a polyorganosiloxane obtainable by curing the curable, high refractive index, organosiloxane composition claimed in any one of claims 1 to 18.

## Patentansprüche

1. Einen hohen Brechungsindex aufweisende, härtbare Polyorganosiloxanmasse zur Verwendung bei der Herstellung von Kontaktlinsen, umfassend
etwa 30 Gew.-% bis etwa 55 Gew.-% eines ersten, eine endständige Vinylgruppe aufweisenden Copolymerharzes mit etwa 80 Mol-% bis etwa 95 Mol-% Dimethylsiloxan und etwa 5 Mol-% bis etwa 20 Mol-% Diphenylsiloxan, wobei das erste, eine endständige Vinylgruppe aufweisende Copolymer ein so hohes Molekulargewicht aufweist, daß das erste, eine endständige Vinylgruppe aufweisende Copolymer eine Viskosität von etwa 0,4 Pa·s (400 cps) bis etwa 2,5 Pas (2500 cps) erhält;
etwa 45 Gew.-% bis etwa 70 Gew.-% eines zweiten, verschiedenen, eine endständige Vinylgruppe aufweisenden Copolymerharzes mit etwa 80 Mol-% bis etwa 95 Mol-% Dimethylsiloxan und etwa 5 Mol-% bis etwa 20 Mol-% Diphenylsiloxan, wobei das zweite, eine endständige Vinylgruppe aufweisende Copolymer ein so hohes Molekulargewicht aufweist, daß das zweite, eine endständige Vinylgruppe aufweisende Copolymer eine Viskosität von etwa 2,5 Pa·s (2500 cps) bis etwa 9,5 Pa·s (9500 cps) erhält;
ein Organohydrogenpolysiloxanvernetzungsmittel in Form von Tetrakis(dimethylsiloxy)silan und
etwa 8 bis etwa 25 Teile Quarzstaub pro 100 Teile Harz.

2. Den hohen Brechungsindex aufweisende, härtbare Polyorganosiloxanmasse nach Anspruch 1, welche zusätzlich einen Platinkatalysator enthält.

3. Den hohen Brechungsindex aufweisende, härtbare Polyorganosiloxanmasse nach Anspruch 1 oder 2, wobei das erste, eine endständige Vinylgruppe aufweisende Polysiloxan die Formel: 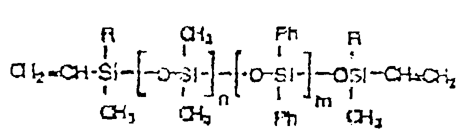 worin bedeuten:
R einen einwertigen organischen Rest, ausgewählt aus der Gruppe -CH₃ und CH=CH₂, und
n + m eine ganze Zahl, die ausreichend hoch ist, daß das erste, eine endständige Vinylgruppe aufweisende Copolymer eine Viskosität von etwa 0,4 Pa·s (400 cps) bis etwa 2,5 Pa·s (2500 cps)
aufweist.

4. Den hohen Brechungsindex aufweisende, härtbare Polyorganosiloxanmasse nach einem der Ansprüche 1 bis 3, wobei das zweite, eine endständige Vinylgruppe aufweisende Polysiloxan die Formel: 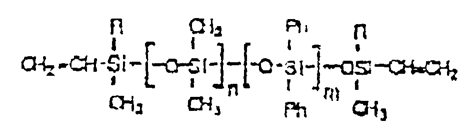 worin bedeuten:
R einen einwertigen organischen Rest, ausgewählt aus der Gruppe -CH₃ und CH=CH₂, und
n + m eine ganze Zahl, die ausreichend hoch ist, daß das zweite, eine endständige Vinylgruppe aufweisende Copolymer eine Viskosität von etwa 4,0 Pas (4000 cps) bis etwa 9,5 Pas (9500 cps)
aufweist.

5. Den hohen Brechungsindex aufweisende, härtbare Polyorganosiloxanmasse nach einem der Ansprüche 1 bis 4, wobei das erste, eine endständige Vinylgruppe aufweisende Polysiloxan etwa 12 Mol-% bis etwa 18 Mol-% Diphenylsiloxan enthält.

6. Den hohen Brechungsindex aufweisende, härtbare Polyorganosiloxanmasse nach einem der Ansprüche 1 bis 5, wobei das zweite, eine endständige Vinylgruppe aufweisende Polysiloxan etwa 12 Mol-% bis etwa 18 Mol-% Diphenylsiloxan enthält.

7. Den hohen Brechungsindex aufweisende, härtbare Polyorganosiloxanmasse nach einem der Ansprüche 1 bis 6, wobei die Viskosität des ersten, eine endständige Vinylgruppe aufweisenden Polysiloxans etwa 0,7 Pas (700 cps) beträgt.

8. Den hohen Brechungsindex aufweisende, härtbare Polyorganosiloxanmasse nach einem der Ansprüche 1 bis 7, wobei die Viskosität des zweiten, eine endständige Vinylgruppe aufweisenden Polysiloxans etwa 5,0 Pas (5000 cps) beträgt.

9. Den hohen Brechungsindex aufweisende, härtbare Polyorganosiloxanmasse nach einem der Ansprüche 1 bis 8, wobei der Quarzstaub einen durchschnittlichen Teilchendurchmesser von etwa 7 nm aufweist.

10. Den hohen Brechungsindex aufweisende, härtbare Polyorganosiloxanmasse nach einem der Ansprüche 1 bis 9, welche zusätzlich eine UV-Licht absorbierende Verbindung enthält.

11. Den hohen Brechungsindex aufweisende, härtbare Polyorganosiloxanmasse nach Anspruch 10, wobei die UV-Licht absorbierende Verbindung aus Vinylhydroxyphenylbenzotriazol besteht.

12. Den hohen Brechungsindex aufweisende, härtbare Polyorganosiloxanmasse nach Anspruch 10, wobei die UV-Licht absorbierende Verbindung aus Siliconhydridhydroxyphenylbenzotriazol besteht.

13. Den hohen Brechungsindex aufweisende, härtbare Polyorganosiloxanmasse zur Verwendung bei der Herstellung von Kontaktlinsen, umfassend
eine Harzgrundlage aus
etwa 48 Gew.-% eines ersten, eine endständige Vinylgruppe aufweisenden Copolymers mit etwa 82 Mol-% bis etwa 88 Mol-% Dimethylsiloxan und etwa 12 Mol-% bis etwa 18 Mol-% Diphenylsiloxan, wobei das erste, eine endständige Vinylgruppe aufweisende Copolymer ein so hohes Molekulargewicht aufweist, daß das erste, eine endständige Vinylgruppe aufweisende Copolymer eine Viskosität von etwa 0,7 Pas (700 cps) erhält und
etwa 52 Gew.-% eines zweiten, verschiedenen, eine endständige Vinylgruppe aufweisenden Copolymers mit etwa 82 Mol-% bis etwa 88 Mol-% Dimethylsiloxan und etwa 12 Mol-% bis etwa 18 Mol-% Diphenylsiloxan, wobei das zweite, eine endständige Vinylgruppe aufweisende Copolymer ein so hohes Molekulargewicht aufweist, daß das zweite, eine endständige Vinylgruppe aufweisende Copolymer eine Viskosität von etwa 5,0 Pas (5000 cps) erhält;
etwa 11 bis etwa 14 Teile Quarzstaub pro 100 (Teile) Harzgrundlage;
bezogen auf die Harzgrundlage etwa 5 bis etwa 50 ppm eines platinhaltigen Katalysators;
etwa 1,5 bis etwa 5 Teile eines Organohydrogenpolysiloxanvernetzungsmittels in Form von Tetrakis(dimethylsiloxan)silan pro 100 (Teile) Harzgrundlage und
etwa 0,1 bis etwa 2 Teil(e) einer UV-Licht absorbierenden Verbindung, ausgewählt aus der Gruppe Hydroxybenzophenone und Hydroxyphenylbenzotriazole pro 100 (Teile) Harz.

14. Den hohen Brechungsindex aufweisende, härtbare Polyorganosiloxanmasse nach Anspruch 13, wobei der Quarzstaub mit Verbindungen, ausgewählt aus der Gruppe Hexamethyldisilazan und 1,3-Divinyltetramethyldisilazan oberflächenbehandelt ist.

15. Den hohen Brechungsindex aufweisende, härtbare Polyorganosiloxanmasse nach Anspruch 13 oder 14, wobei das Vinylhydroxyphenylbenzotriazol aus 2-[5-Chlor-2H-benzotriazol-2-yl]-6-[1,1-dimethylethyl]-4-[2-propenyloxypropyl]phenol besteht.

16. Den hohen Brechungsindex aufweisende, härtbare Polyorganosiloxanmasse nach Anspruch 15, wobei das 2-[5-Chlor-2H-benzotriazol-2-yl]-6-[1,1-dimethylethyl]-4-[2-propenyloxypropyl]phenol mit Tetrakis(dimethylsiloxy)silan hydrosilyliert ist.

17. Den hohen Brechungsindex aufweisende, härtbare Polyorganosiloxanmasse nach Anspruch 15, wobei das 2-[5-Chlor-2H-benzotriazol-2-yl]-6-[1,1-dimethylethyl]-4-[2-propenyloxypropyl]phenol mit einem Terpolymer von Diphenylsiloxan, Dimethylsiloxan und Methylhydrogensiloxan hydrosilyliert ist.

18. Den hohen Brechungsindex aufweisende, härtbare Polyorganosiloxanmasse nach einem der Ansprüche 13 bis 17, wobei der Quarzstaub eine durchschnittliche Teilchengröße von etwa 7 nm aufweist.

19. Eine elastomere, optisch klare, einen höhen Brechungsindex aufweisende Linse mit hervorragender Erholungsfähigkeit der optischen Auflösung nach dem Falten bzw. Umknicken, umfassend ein Polyorganosiloxan, das durch Härten der härtbaren, einen hohen Brechungsindex aufweisenden Organosiloxanmasse nach einem der Ansprüche 1 bis 18 erhältlich ist.

## Revendications

1. Composition de polyorganosiloxane durcissable d'indice de réfraction élevé utile pour fabriquer des lentilles intraoculaires, ladite composition comprenant :
d'environ 30 % en poids à environ 55 % en poids d'une première résine copolymère à terminaison vinyle ayant d'environ 80 % en moles à environ 95 % en moles de diméthylsiloxane et d'environ 5 % en moles à environ 20 % en moles de diphénylsiloxane, ledit premier copolymère à terminaison vinyle ayant une masse moléculaire suffisante pour donner une viscosité du premier copolymère à terminaison vinyle d'environ 0,4 Pa.s (400 cps) à environ 2,5 Pa.s (2500 cps)
d'environ 45 % en poids à environ 70 % en poids d'une deuxième résine copolymère différente à terminaison vinyle ayant d'environ 80 % en moles à environ 95 % en moles de diméthylsiloxane et d'environ 5 % en moles à environ 20 % en moles de diphénylsiloxane, ledit deuxième copolymère à terminaison vinyle ayant une masse moléculaire suffisante pour donner une viscosité du deuxième copolymère à terminaison vinyle d'environ 2,5 Pa.s (2500 cps) environ 9,5 Pa.s (9500 cps) ;
un réactif de réticulation organohydrogéno-polysiloxane de tétrakis(diméthylsiloxy)silane ; et
d'environ 8 à environ 25 parties de charge de poudre de silice pour 100 parties de résine.

2. Composition de polyorganosiloxane durcissable d'indice de réfraction élevé selon la revendication 1, comprenant en outre un catalyseur au platine.

3. Composition de polyorganosiloxane durcissable d'indice de réfraction élevé selon la revendication 1 ou 2, dans laquelle ledit premier polysiloxane à terminaison vinyle a la formule : 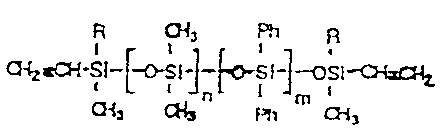 où R est un radical organique monovalent choisi dans le groupe constitué par -CH₃, et CH=CH₂, et n + m est un entier suffisant pour donner ladite viscosité du premier copolymère à terminaison vinyle d'environ 0,4 Pa.s (400 cps) à environ 2,5 Pa.s (2500 cps).

4. Composition de polyorganosiloxane durcissable d'indice de réfraction élevé selon l'une quelconque des revendications 1 à 3, dans laquelle ledit deuxième polysiloxane à terminaison vinyle a la formule : 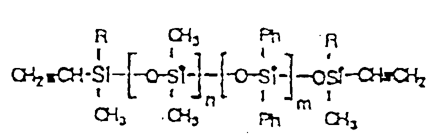 où R est un radical organique monovalent choisi dans le groupe formé par -CH₃, et CH=CH₂, et n + m est un entier suffisant pour donner ladite viscosité du premier copolymère à terminaison vinyle d'environ 4,0 Pa.s (4000 cps) à environ 9,5 Pa.s (9500 cps).

5. Composition de polyorganosiloxane durcissable d'indice de réfraction élevé selon l'une quelconque des revendications 1 à 4, dans laquelle ledit deuxième polysiloxane à terminaison vinyle comprend environ 12 % en moles à environ 18 % en moles de diphénylsiloxane.

6. Composition de polyorganosiloxane durcissable d'indice de réfraction élevé selon l'une quelconque des revendications 1 à 5, dans laquelle ledit deuxième polysiloxane à terminaison vinyle comprend environ 12 % en moles à environ 18 % en moles de diphénylsiloxane.

7. Composition de polyorganosiloxane durcissable d'indice de réfraction élevé selon l'une quelconque des revendications 1 à 6, dans laquelle ledit premier polysiloxane à terminaison vinyle a une viscosité d'environ 0,7 Pa.s (700 cps).

8. Composition de polyorganosiloxane durcissable d'indice de réfraction élevé selon l'une quelconque des revendications 1 à 7, dans laquelle ledit deuxième polysiloxane à terminaison vinyle a une viscosité d'environ 5,0 Pa.s (5000 cps).

9. Composition de polyorganosiloxane durcissable d'indice de réfraction élevé selon l'une quelconque des revendications 1 à 8, dans laquelle ladite poudre de silice a un diamètre de particules moyen d'environ 7 nanomètres.

10. Composition de polyorganosiloxane durcissable d'indice de réfraction élevé selon l'une quelconque des revendications 1 à 9, comprenant en outre un composé absorbant les ultraviolets.

11. Composition de polyorganosiloxane durcissable d'indice de réfraction élevé selon la revendication 10, dans laquelle ledit composé absorbant les ultraviolets est le vinyl hydroxyphénylbenzotriazole.

12. Composition de polyorganosiloxane durcissable d'indice de réfraction élevé selon la revendication 10, dans laquelle ledit composé absorbant les ultraviolets est l' hydrure de silicone-hydroxyphénylbenzotriazole.

13. Composition de polyorganosiloxane durcissable d'indice de réfraction élevé utile pour fabriquer des lentilles intraoculaires, ladite composition comprenant : une résine à base d':
environ 48 % en poids d'un premier copolymère à terminaison vinyle ayant d'environ 82 % en moles à environ 88 % en moles de diméthylsiloxane et d'environ 12 % en moles à environ 18 % en moles de diphénylsiloxane, ledit premier copolymère à terminaison vinyle ayant une masse moléculaire suffisante pour donner une viscosité du premier copolymère à terminaison vinyle d'environ 0,7 Pa.s (700 cps), et
environ 52 % en poids à environ 70 % en poids d'un deuxième copolymère à terminaison vinyle différent ayant d'environ 82 % en moles à environ 88 % en moles de diméthylsiloxane et d'environ 12 % en moles à environ 18 % en moles de diphénylsiloxane, ledit deuxième copolymère à terminaison vinyle ayant une masse moléculaire suffisante pour donner une viscosité du deuxième copolymère à terminaison vinyle d'environ 5,0 Pa.s (5000 cps) ;
d'environ 11 à environ 14 parties de poudre de silice pour 100 parties de résine de base ;
d'environ 5 à environ 50 parties de catalyseur contenant du platine par million de parties de résine de base ;
d'environ 1,5 à environ 5 parties pour 100 parties de résine de base de réactif de réticulation organohydrogéne-polysiloxane de tétrakis(diméthylsiloxy)silane ; et
d'environ 0,1 à environ 2 parties pour 100 parties de résine de base de composé absorbant les ultraviolets choisi dans l'ensemble formé par les hydroxybenzophénones et les hydroxyphénylbenzotriazoles.

14. Composition de polyorganosiloxane durcissable d'indice de réfraction élevé selon la revendication 13, dans laquelle ladite poudre de silice est traitée en surface avec des composés choisis dans le groupe formé par l'hexaméthylsilazane et le 1,3-divinyltétraéthyldisilazane.

15. Composition de polyorganosiloxane durcissable d'indice de réfraction élevé selon la revendication 13 ou 14, dans laquelle ledit hydroxyphénylbenzotriazole est le 2-[5-chlore-2H-benzotriazol-2-yl]-6-[1,1-diméthyléthyl]4-[2-propényloxypropyl]phénol.

16. Composition de polyorganosiloxane durcissable d'indice de réfraction élevé selon la revendication 15, dans laquelle ledit 2-[5-chloro-2H-benzotriazol-2-yl]-6-[1,1-diméthyléthyl]-4-[2-propénylexypropyl]phénol est hydrosilylé avec du tétrakis(diméthylsiloxy)silane.

17. Composition de polyorganosiloxane durcissable d'indice de réfraction élevé selon la revendication 15, dans laquelle ledit 2-[5-chloro-2H-benzotriazol-2-yl]-6-[1,1-diméthyléthyl]-4-[2-[propényloxypropyl]phénol est hydrosilylé avec un terpolymère de diphénylsiloxane, diméthylsiloxane et méthylhydrogénosiloxane.

18. Composition de polyorganosiloxane durcissable d'indice de réfraction élevé selon l'une quelconque des revendication 13 à 17, dans laquelle ladite poudre de silice à une taille de particules moyenne d'environ 7 nanomètres.

19. Lentille élastomère à indice de réfraction élevé optiquement transparente ayant une récupération de résolution optique excellente après pliage, ladite lentille comprenant un polyorganosiloxane pouvant être obtenu par durcissement de la composition d'organosiloxane durcissable d'indice de réfraction élevé selon l'une quelconque des revendications 1 à 18.
